(19) Europäisches Patentamt

European Patent Office

Office européen des brevets

(11) EP 0 511 612 B1

(12) **EUROPÄISCHE PATENTSCHRIFT**

(45) Veröffentlichungstag und Bekanntmachung des Hinweises auf die Patenterteilung:
**04.12.1996 Patentblatt 1996/49**

(51) Int. Cl.$^6$: **C07C 17/389**, C07C 19/08

(21) Anmeldenummer: **92107140.3**

(22) Anmeldetag: **27.04.1992**

(54) **Verfahren zur Reinigung von Fluorchlorkohlenwasserstoffen**

Method for the purification of chlorofluorohydrocarbons

Procédé pour l'épuration de fluorochlorohydrocarbures

(84) Benannte Vertragsstaaten:
**DE ES FR GB GR IT NL**

(30) Priorität: **27.04.1991 DE 4113907**

(43) Veröffentlichungstag der Anmeldung:
**04.11.1992 Patentblatt 1992/45**

(73) Patentinhaber: **HOECHST AKTIENGESELLSCHAFT 65926 Frankfurt am Main (DE)**

(72) Erfinder: **Jansen, Rolf-Michael, Dr. W-6233 Kelkheim (Taunus) (DE)**

(56) Entgegenhaltungen:
**EP-A- 0 199 212        EP-A- 0 370 688**
**US-A- 3 696 156        US-A- 4 906 796**

**Beschreibung**

Die vorliegende Erfindung betrifft ein Verfahren zur Entfernung olefinischer Verunreinigungen aus wasserstoffhaltigen Fluorchlorkohlenwasserstoffen. Wasserstoffhaltige Fluorchlorkohlenwasserstoffe (H-FCKW) werden als Ersatzstoffe für die ozongefährdenden vollhalogenierten, d. h. wasserstofffreien Fluorchlorkohlenwasserstoffe (FCKW) verwendet. Um die H-FCKW in Bereichen wie Verschäumung und Kältetechnik oder als Aerosole anwenden zu können, müssen jedoch unerwünschte Nebenprodukte, die bei der Synthese, zum Teil im Spurenbereich, entstehen können, quantitativ entfernt werden. Bei diesen Nebenprodukten handelt es sich hauptsächlich um Olefine, die teilweise toxisch sind.

Eine bekannte Methode zur Entfernung mancher ungesättigter Verunreinigungen stellt die Adsorption an Aktivkohle oder Zeolithen dar. So zeigt beispielsweise US-PS 4 940 825 ein Verfahren zur Entfernung von Dichloracetylen aus R 141b (1,1-Dichlor-1-fluorethan) oder Vinylchlorid durch Adsorption bei - 20 °C bis + 60 °C an Aktivkohle mit einer Porengröße von 4,2 - 4,8 Angström. US-PS 4 922 044 beschreibt, daß Dichlorethylen und Bromchlormethan bei 10 bis 150 °C durch Adsorption an Calcium-Chabazit aus Chloroform entfernt werden. Gemäß US-PS 4 906 796 wird R 1122 (Difluorchlorethylen) durch Adsorption bei - 10 bis 100 °C an einem Zeolith oder Aktivkohle mit einem Porenradius von 3,8 - 4,8 Angström aus R 134a (1,1,1,2-Tetrafluorethan) abgetrennt. In der Regel lassen sich aber Olefine mit mehr als zwei C-Atomen auf diesem Wege nicht von H-FCKW der Ethanreihe abtrennen. Ein weiterer Nachteil ist, daß die im Adsorbens stark angereicherten, eventuell hochtoxischen Nebenprodukte anschließend unter hohem Aufwand entsorgt werden müssen.

Eine andere Methode wird in US-PS 3 696 156 beschrieben, bei der olefinische Verunreinigungen aus FCKW entfernt werden, indem die verunreinigten FCKW bei 180 - 250 °C über Aluminiumoxid geleitet werden, das mit Alkali- oder Erdalkalihydroxiden behandelt wurde. Eine ähnliche Methode lehrt EP-A - 370 688, bei der die mit Olefinen verunreinigten FCKW bei Temperaturen bis zu 300 °C, vorzugsweise unterhalb etwa 250 °C über ein amorphes Metalloxid geleitet werden. Nachteilig ist bei beiden Verfahren, daß die verwendeten Feststoffe nur mit großem Aufwand in einer gleichbleibenden Qualität hergestellt werden können. Außerdem werden manche Verunreinigungen nur in geringem Maße abgebaut.

Gegenstand der Erfindung ist ein Verfahren zur Entfernung olefinischer Verunreinigungen der allgemeinen Formel $C_mH_nF_pCl_q$ , wobei m = 2 - 3, n = 1 - 4, p = 1 - 6, q = 0 - 4 und n + p + q = 2m ist, oder von 1,1,1,4,4,4-Hexafluor-2-chlorbuten-2 aus wasserstoffhaltigen Fluorchlorkohlenwasserstoffen (H-FCKW) der allgemeinen Formel $C_aH_bF_cCl_d$ , wobei a = 2 - 3, b = 1 - 5, c = 1 - 7, d = 0 - 4 und b + c + d = 2a + 2 ist, dadurch gekennzeichnet, daß man die verunreinigten Fluorchlorkohlenwasserstoffe in der Gasphase bei Temperaturen von 200 bis 400 °C und Drücken von 1 bis 50 bar über einen Zeolith leitet, dem in der wasserfreien und templatfreien Form die allgemeine Formel Z · $Al_2O_3$ · x $(SiO_2)$ zukommt, in welcher Z = H, $NH_4$, $M(I)_2O$, M(II)O oder $M(III)_2O_3$ ist, worin M(I) für ein Alkalimetall, M(II) für ein Erdalkalimetall und M(III) für ein seltenes Erdmetall der Ordnungszahl 57 - 71 im Periodischen System der Elemente steht und x eine Zahl von 2 bis 2000 bedeutet.

Als H-FCKW kommen vor allem in Frage:
1,1,1-Trifluor-2-chlorethan (R 133a), 1,1,1-Trifluor-2,2-dichlorethan (R 123), 1,1,1,2-Tetrafluorethan (R 134a ), 1,1,1,2,2-Pentafluorethan (R 125) und 1,1,1,2,3,3,3-Heptafluorpropan (R 227).

Übliche olefinische Verunreinigungen sind beispielsweise:
1,1-Difluorchlorethen, 1,1-Difluordichlorethen, 1,2-Difluorethen, 1,1,2-Trifluorchlorethen, 1H-Pentafluorpropen, 2H-Pentafluorpropen, 1,1,1,4,4,4-Hexafluor-2-chlorbuten-2 und 2-Trifluormethyl-3,3,3-trifluorpropen-1.

Die verunreinigten H-FCKW werden vorzugweise bei 250 - 350 °C, insbesondere bei 290 - 310 °C über den Zeolith geleitet. Der Druck beträgt dabei vorzugsweise 1 - 25 bar.

Der Zeolith befindet sich dabei beispielsweise in einem beheizbaren Glasrohr, wobei das Verhältnis von Rohrdurchmesser zu Schütthöhe des Zeoliths vorzugsweise 1 : 5 bis 1 : 15 beträgt.

Der gasförmige, verunreinigte H-FCKW kann über eine kurze Vorheizstrecke auf die gewählte Reaktionstemperatur gebracht werden. Höhersiedende H-FCKW, wie R 123, müssen vorher in einem separaten Verdampfer in die Gasphase überführt werden.

Die Gasgeschwindigkeit bei der Reaktion beträgt vorzugsweise 2 - 50 l/h. Weiter ist es bevorzugt, die verunreinigten H-FCKW in einem inerten Trägergasstrom, wie $N_2$, über den Zeolith zu leiten.

Der eingesetzte Zeolith hat in der wasserfreien und templatfreien Form die allgemeine Formel Z · $Al_2O_3$ · x $(SiO_2)$. Dabei ist Z = H, $NH_4$, $M(I)_2O$, M(II)O oder $M(III)_2O_3$, worin M(I) ein Alkalimetall, M(II) ein Erdalkalimetall und M(III) ein seltenes Erdmetall der Ordnungszahl 57 - 71 bedeutet. Vorzugsweise ist M(I) = Li, Na oder K, M(II) = Ca, Ba oder Sr und M(III) = La oder Ce. Weiter ist x eine Zahl von 2 bis 2000, vorzugsweise von 2 bis 200, insbesondere von 2 bis 50. Die genannten Zeolithe sind beschrieben in Donald W. Breck, Zeolite Molecular Sieves, 1974, Wiley and Sons.

Besonders bevorzugt ist der Einsatz von H-ZSM 5 (US-PS 3 702 886), A-Zeolith (US-PS 2 882 243), X-Zeolith (US-PS 2 882 244) und Y-Zeolith (US-PS 3 130 007). Besonders geeignete Formen von A-Zeolith sind das 5-Angström-Molsieb und vor allem das 10-Angström-Molsieb.

Der Zeolith kann sowohl als Pulver wie auch in Gestalt von Formkörpern, z. B. Kugeln oder Zylindern verwendet werden. Beim Einsatz: von Pulver wird der Strömungswiderstand vorzugsweise durch Vermischen mit Glas- oder Metallkörpern, z. B. Raschigringen oder Metallwendeln, reduziert, um geeignete Strömungsgeschwindigkeiten zu erreichen.

Der Zeolith läßt sich, wenn notwendig, mit Luft oder Sauerstoff bei erhöhten Temperaturen wieder regenerieren.

Vorzugsweise wird den verunreinigten H-FCKW kontinuierlich 0,5 bis 10 Vol.-% Sauerstoff oder Luft zugegeben, ehe man sie über den Zeolith leitet; dadurch wird die Verkokung sehr gering gehalten.

Die olefinischen Verunreinigungen werden offenbar beim erfindungsgemäßen Verfahren ganz oder weitestgehend abgebaut, im Unterschied zu den oben genannten Adsorptionsverfahren, bei denen die Verunreinigungen anschließend vom Adsorbens entfernt und beseitigt werden müssen.

Die folgenden Beispiele sollen die Erfindung näher erläutern.

Beispiele 1 - 4:

50 ml 1,1-Dichlor-2,2,2-trifluorethan (R 123), das 1500 ppm 1,1,1,4,4,4-Hexafluor-2-chlor-buten-2 (R 1326) enthielt, wurden bei 300 °C im Stickstoffstrom, dessen Strömungsgeschwindigkeit (25,2 l/h) mit einem Massendurchflußregler gesteuert wurde, durch ein Glasrohr (Innendurchmesser = 20 mm, Länge = 100 cm), das mit ca. 50 g Zeolith (Schütthöhe ca. 30 cm) gefüllt war, geschickt und anschließend kondensiert. Der Gehalt von R 1326 im R 123 wurde gaschromatographisch bestimmt. Die Ergebnisse sind in Tabelle 1 zusammengefaßt.

Tabelle 1

| Beispiel | Zeolith | Kondensat [ml] | Reaktionsdauer [h] | Gehalt R 1326 [ppm] | Leistung [ml/h] |
|---|---|---|---|---|---|
| 1 | H-ZSM-5 | 46 | 4 | 45 | 12.5 |
| 2 | Y-Zeolith | 42 | 2 | unterhalb der Nachweisgrenze | 25 |
| 3 | 5-Å-Molsieb | 40 | 3 | 258 | 16.6 |
| 4 | 10-Å-Molsieb | 41 | 3 | unterhalb der Nachweisgrenze | 16.6 |

Vergleichsbeispiele 1 - 3:

Zum Vergleich wurden die Experimente der Beispiele 1 - 4 anstatt mit Zeolith mit $SiO_2$, $\gamma$-$Al_2O_3$ und Aktivkohle (spez. Oberfläche nach BET: 1250 $m^2$/g) durchgeführt. Die Ergebnisse sind in Tabelle 2 dargestellt.

Tabelle 2

| Vergleichsbeispiel | Reaktorfüllung | Kondensat [ml] | Reaktionsdauer [h] | Gehalt R 1326 [ppm] | Leistung [ml/h] |
|---|---|---|---|---|---|
| 1 | Aktivkohle | 44 | 3 | 1343 | 16.6 |
| 2 | $SiO_2$ | 41 | 4 | 2640 | 12.5 |
| 3 | $\gamma$-$Al_2O_3$ | 41 | 4 | 1290 | 12.5 |

**Patentansprüche**

1. Verfahren zur Entfernung olefinischer Verunreinigungen der allgemeinen Formel $C_mH_nF_pCl_q$, wobei m = 2 - 3, n = 1 - 4, p = 1 - 6, q = 0 - 4 und n + p + q = 2m ist, oder von 1,1,1,4,4,4-Hexafluor-2-chlorbuten-2 aus wasserstoffhaltigen Fluorchlorkohlenwasserstoffen der allgemeinen Formel $C_aH_bF_cCl_d$, wobei a = 2 - 3, b = 1 - 5, c = 1 - 7, d = 0 - 4 und b + c + d = 2a + 2 ist, dadurch gekennzeichnet, daß man die verunreinigten Fluorchlorkohlenwasserstoffe in der Gasphase bei Temperaturen von 200 bis 400 °C und Drücken von 1 bis 50 bar über einen Zeolith leitet, dem in der wasserfreien und templatfreien Form die allgemeine Formel $Z \cdot Al_2O_3 \cdot x (SiO_2)$ zukommt, in welcher Z = H, $NH_4$, $M(I)_2O$, $M(II)O$ oder $M(III)_2O_3$ ist, worin M(I) für ein Alkalimetall, M(II) für ein Erdalkalimetall und M(III) für ein seltenes Erdmetall der Ordnungszahl 57 - 71 im Periodischen System der Elemente steht und x eine Zahl von 2 bis 2000 bedeutet.

2. Verfahren nach Anspruch 1, dadurch gekennzeichnet, daß man bei 250 bis 350 °C arbeitet.

3. Verfahren nach Anspruch 1, dadurch gekennzeichnet, daß man bei 290 bis 310 °C arbeitet.

4. Verfahren nach einem der Ansprüche 1 bis 3, dadurch gekennzeichnet, daß man die verunreinigten Fluorchlorkohlenwasserstoffe in einem Trägergasstrom über den Zeolith leitet.

5. Verfahren nach einem der Ansprüche 1 bis 4, dadurch gekennzeichnet, daß man den verunreinigten Fluorchlorkohlenwasserstoffen kontinuierlich 0.5 - 10 Vol.-% Sauerstoff oder Luft zudosiert, bevor man sie über den Zeolith leitet.

6. Verfahren nach einem der Ansprüche 1 bis 5, dadurch gekennzeichnet, daß man als Zeolith H-ZSM 5, Zeolith A, Zeolith X oder Zeolith Y einsetzt.

7. Verfahren nach einem der Ansprüche 1 bis 6, dadurch gekennzeichnet, daß man olefinische Verunreinigungen aus einem der wasserstoffhaltigen Fluorchlorkohlenwasserstoffe 1,1,1-Trifluor-2-chlorethan (R 133a), 1,1,1-Trifluor-2,2-dichlorethan (R 123), 1,1,1,2-Tetrafluorethan (R 134a), 1,1,1,2,2-Pentafluorethan (R 125) oder 1,1,1,2,3,3,3-Heptafluorpropen (R 227) entfernt.

**Claims**

1. A process for removing olefinic impurities of the formula $C_mH_nF_pCl_q$ where m = 2 - 3, n = 1 - 4, p = 1 - 6, q = 0 - 4 and n + p + q = 2m , or 1,1,1,4,4,4,-hexafluoro-2-chloro-2-butene from hydrogen-containing chlorofluorocarbons of the formula $C_aH_bF_cCl_d$ where a = 2 - 3, b = 1 - 5, c = 1 - 7, d = 0 - 4 and b + c + d = 2a + 2 , which comprises passing the contaminated chlorofluorocarbons in the gas phase at temperatures of 200 to 400°C and pressures of from 1 to 50 bar over a zeolite which, in the anhydrous and template-free form, has the formula $Z \cdot Al_2O_3 \cdot x(SiO_2)$ in which Z = H, $NH_4$, $M(I)_2O$, M(II)O or $M(III)_2O_3$ where M(I) is an alkali metal, M(II) is an alkaline earth metal and M(III) is a rare earth metal having an atomic number from 57 - 71 in the Periodic Table of the Elements, and x is a number from 2 to 2000.

2. The process as claimed in claim 1, which is carried out at from 250 to 350°C.

3. The process as claimed in claim 1, which is carried out at from 290 to 310°C.

4. The process as claimed in any of claims 1 to 3, wherein the contaminated chlorofluorocarbons are passed over the zeolite in a stream of carrier gas.

5. The process as claimed in any of claims 1 to 4, wherein from 0.5 to 10 % by volume of oxygen or air is continuously metered into the contaminated chlorofluorocarbons before they are passed over the zeolite.

6. The process as claimed in any of claims 1 to 5, wherein the zeolite employed is H-ZSM 5, zeolite A, zeolite X or zeolite Y.

7. The process as claimed in any of claims 1 to 6, wherein olefinic impurities are removed from one of the hydrogen-containing chlorofluorocarbons 1,1,1-trifluoro-2-chloroethane (R 133a), 1,1,1-trifluoro-2,2-dichloroethane (R 123), 1,1,1,2-tetrafluoroethane (R 134a), 1,1,1,2,2-pentafluoroethane (R 125) or 1,1,1,2,3,3,3-heptafluoropropene (R 227).

**Revendications**

1. Procédé pour l'élimination des impuretés oléfiniques de formule générale $C_mH_nF_pCl_q$, m = 2 - 3, n = 1 - 4, p = 1 - 6, q = 0 - 4 et n + p + q = 2m ou du 1,1,1,4,4,4-hexafluoro-2-chlorobutène-2, à partir des hydrocarbures fluorochlorés hydrogénés, de formule générale $C_aH_bF_cCl_d$, a = 2 - 3, b = 1 - 5, c = 1 - 7, d = 0 - 4 et b + c + d = 2a + 2 , caractérisé en ce qu'on conduit les hydrocabures fluorochlorés en phase gazeuse souillés, à des températures de 200 à 400°C et des pressions de 1 à 50 bars sur une zéolithe, qui sous forme anhydre et sans motif répond à la formule générale $Z \cdot Al_2O_3 \cdot x (SiO_2)$ dans laquelle Z = H, $NH_4$, $M(I)_2O$, M(II)O ou $M(III)_2O_3$, où M(I) est un métal alcalin, M(II) est un métal alcalino-terreux et M(III) est un métal de terres rares ayant un nombre atomique de 57 à 71 du Système Périodique des Eléments et x est un nombre de 2 à 2000.

2. Procédé selon la revendication 1, caractérisé en ce qu'on travaille à des températures de 250 à 350°C.

3. Procédé selon la revendication 1, caractérisé en ce qu'on travaille à des températures de 290 à 310°C.

4. Procédé selon l'une des revendications 1 à 3, caractérisé en ce qu'on conduit les hydrocarbures fluorochlorés souillés dans un courant de gaz porteur sur la zéolithe.

5. Procédé selon l'une des revendications 1 à 4, caractérisé en ce qu'on ajoute progressivement aux hydrocarbures fluorochlorés souillés de 0,5 à 10% en volume d'oxygène ou d'air, avant qu'on les conduise sur la zéolithe.

6. Procédé selon l'une des revendications 1 à 5, caractérisé en ce qu'on utilise en tant que zéolithe H-ZSM 5, A-Zolith, X-Zeolith ou Y-Zeolith.

7. Procédé selon l'une des revendications 1 à 6, caractérisé en ce qu'on élimine les impuretés oléfiniques à partir des hydrocarbures fluorochlorés hydrogénés le 1,1,1-trifluoro-2-chloréthane (R 133a), le 1,1,1-trifluoro-2,2-dichloréthane (R 123), le 1,1,1,2-tétrafluoréthane (R 134a), le 1,1,1,2,2-pentafluoréthane (R 125) et le 1,1,1,2,3,3,3-heptafluoropropane (R 227).